(19) Europäisches Patentamt
European Patent Office
Office européen des brevets

(11) Veröffentlichungsnummer: **0 256 385 B1**

(12) **EUROPÄISCHE PATENTSCHRIFT**

(45) Veröffentlichungstag der Patentschrift: **15.11.95**

(51) Int. Cl.$^6$: **A61K 31/16**

(21) Anmeldenummer: **87111029.2**

(22) Anmeldetag: **30.07.87**

(54) **Verwendung von 15-Deoxyspergualin als Arzneimittel.**

(30) Priorität: **02.08.86 DE 3626306**

(43) Veröffentlichungstag der Anmeldung:
**24.02.88 Patentblatt 88/08**

(45) Bekanntmachung des Hinweises auf die
Patenterteilung:
**15.11.95 Patentblatt 95/46**

(84) Benannte Vertragsstaaten:
**AT BE CH DE ES FR GB GR IT LI LU NL SE**

(56) Entgegenhaltungen:
**EP-A- 0 094 632
EP-A- 0 181 592
WO-A-87/02583
DE-A- 3 508 033
US-A- 4 525 299**

**THE MERCK MANUAL, 14. Ed. 1982; R. BER-KOW M.D., Merck Sharp & Dohme Research Lab., Rahway, N.J., US, Seiten 328-342**

**THE MERCK MANUAL, 14. Ed. 1982; R. BER-KOW M.D., Merck Sharp & Dohme Research Lab., Rahway, N.J., US, Seiten 1165-1166**

(73) Patentinhaber: **BEHRINGWERKE Aktiengesell-schaft
Postfach 1140
D-35001 Marburg (DE)**

(72) Erfinder: **Dickneite, Gerhard, Dr.
Zum Neuen Hieb 31
D-3550 Marburg (DE)**
Erfinder: **Schorlemmer, Hans-Ulrich, Dr.
Am Kirschenwald 2
D-3550 Marburg (DE)**
Erfinder: **Kraemer, Hans Peter, Dr.
Birkenweg 16
D-3550 Marburg (DE)**
Erfinder: **Sedlacek, Hans Harald, Dr.
Sonnenhang 3
D-3550 Marburg (DE)**

DORLAND'S ILLUSTRATED MEDICAL DICTIO-NARY, Ed. 26, 1985; W.B. SAUNDERS CO., Philadelphia, US

THE MERCK MANUAL, 14. Ed.,1982; R. BER-KOW M.D., Merck Sharp & Dohme Research Laboratories, Rahway, N.J., US, Seiten 1107-1110

THE MERCK MANUAL, 14. Ed., 1982; R. BER-KOW M.D., Merck Sharp & Dohme Research Laboratories, Rahway, N.J., US, Seiten 1137-1146

BEHRING INST. MITT., Nr. 80, Juni 1986, Sei-ten 93-102; G. DICKNEITE et al.: "The influen-ce of (+/-)-15-deoxyspergualin on experi-mental transplantation and its immunophar-macological mode of action"

PROC. ANN. MEETING AM. ASSOC. CANCER RES., Band 27, März 1986, Seite 418, Zusam-menfassung 1660; R.H. WELTMAN et al.: "The toxicologic evaluation of 15-deoxyspergua-ling HCl (NSC-356894) in CD2F1 mice, fi-scher 344 rats, and beagle dogs"

B.G. BOYSEN et al.: "The toxicologic evalua-tion of 15-deoxyspergualin HC1 (NSC-356894) after continuous intravenous infu-sion in beagle dogs", Seite 418, Zusammen-fassung 1661

EUR. SURG. RES., Band 19, Nr. 3, Februar 1987, Seiten 129-134, S. KARGER AG, Basel,

CH; J.C. THIES et al.: "Prologation of graft survival in allogeneic pancreas and liver transplantation by (-)15-deoxyspergualin"

THE JOURNAL OF ANTIBIOTICS, Band 40, Nr. 7, Juni 1987, Seiten 1062-1064; K. NEMOTO et al.: "Blastogenic responses and the release of interleukins 1 and 2 by spleen cells obtai-ned from rat skin allograft recipients admini-stered with 15-deoxyspergualin"

THE JOURNAL OF ANTIBIOTICS, Band 40, Nr. 7, Juni 1987, Seiten 1029-1035; W.E.G. MÜL-LER et al.: "Deoxyspergualin, a potent antitu-mor agent: further studies on the cytobiolo-gical mode of action"

THE JOURNAL OF ANTIBIOTICS, Band 40, Nr. 8, August 1987, Seiten 1193-1194; K. NEMOTO et al.: "Suppression of experimental allergic encephalomyelitis in guinea pigs ba sper-gualin and 15-deoxyspergualin"

THE JOURNAL OF ANTIBIOTICS, Band 42, Nr. 2, Februar 1989, Seiten 312-316; K. NEMOTO et al.: "Effects of deoxyspergualin on hema-topoiesis: studies of murine hematopoietic progenitor cell and peripheral blood cell le-vels"

IMMUN. INFEKT., Band 16, Nr. 1, 1988, Seiten 23-24; G. DICKNEITE et al.: "Präklinische Un-tersuchungen mit 15-deoxyspergualin bei experimenteller Graft-versus-host-Erkran-kung (GvHD)"

**Beschreibung**

Die Erfindung betrifft die Verwendung von 15-Deoxyspergualin zur Herstellung eines Arzneimittels für die Behandlung von degenerativen Erkrankungen des Zentralnervensystems.

15-Deoxyspergualin wurde von Prof. Umezawa und Mitarbeitern gefunden (EP 83 104 712.1). Seine antitumorale Wirksamkeit und immunsuppressive Eigenschaft wurde beschrieben (EP 83 104 712.1 und 85 114 042.6).

Es wurde nun überraschend gefunden, daß die Substanz neben ihrer Wirkung in der Immunsuppression dosisabhängig einen stimulierenden Effekt auf die koloniebildenden Zellen des Knochenmarkes hat, somit einen therapeutischen Effekt auf verschiedene degenerative Erkrankungen bereits dann besitzt, wenn ihre immunsuppressive Wirkung vernachlässigbar ist.

Zu den degenerativen Erkrankungen, bei denen Substanzen mit stimulierender Eigenschaft auf das Knochenmark wirken, gehören Erkrankungen des Knochenmarkes, des weiteren degenerative Erkrankungen des Stütz- und Bindegewebes (Gupta et al. Arthritis Rheum. 118, 179 (1975) und Amer. J. Med. 61, 29 (1976)).

Es wurde nun insbesondere gefunden, daß 15-Deoxyspergualin einen therapeutischen Einfluß auf die degenerative Erkrankung des Zentralnervensystems (ZNS) besitzt, wobei die angewandte Dosierung keine immunsuppressive Wirksamkeit hatte.

Gegenstand der Erfindung ist demzufolge die Verwendung Von 15-Deoxyspergualin zur Herstellung eines Arzneimittels, das die koloniebildenden Zellen des Knochenmarks stimuliert, zur Therapie von degenerativen Erkrankungen des Zentralnervensystems.

Die multiple Sklerose ist eine chronisch degenerative Erkrankung des Zentralnervensystems, deren Ursache weitestgehend unbekannt ist. Als experimentelles Modell der multiplen Sklerose gilt die experimentelle allergische Enzephalomyelitis (EAE) der Ratte, die durch die Gabe von Myelin, einer Substand des Zentralnervensystems, ausgelöst wird. Die Erkrankung beginnt mit der Lähmung der Extremitäten und führt schließlich zum Tod der Tiere. Die Verabreichung von Immunsuppressiva hat in derartigen Therapieversuchen bei der ausgebrochenen Erkrankung bisher nur eine eingeschränkte Wirksamkeit gezeigt. Überraschenderweise zeigt 15-Deoxyspergualin bei geeigneter Dosierung deutliche Wirksamkeit.

Die wirksame Untergrenze der Dosierung von Deoxyspergualin liegt hierfür etwa im Bereich von 0,01 mg/kg Körpergewicht bei parenteraler Verabreichung. Sie wird durch die Toxizität der Substanz limitiert, die bei 13 mg/kg liegt.

Gegenstand der Erfindung ist die Verwendung von 15-Deoxyspergualin, vorzugsweise in Form des preiswerteren Racemates, insbesondere jedoch als (-) -15-Deoxyspergualin zur Herstellung eines Arzneimittels für die therapeutische Behandlung von degenerativen Erkrankungen des Zentralnervensystems, wobei das Arzneimittel geeignet ist, den Wirkstoff in einer Dosierung von 0,01 mg bis 13 mg pro kg Körpergewicht zu applizieren. Für die orale oder parenterale, speziell intravenöse Verabreichung, kommen an sich bekannte physiologisch verträgliche wässrige Lösungen oder Suspensionen des Wirkstoffes in einem pharmazeutisch verträglichen Vektor, vorzugsweise Pflanzenöl, wie Erdnußöl oder Sesamöl sowie alkoholische Lösungen des Wirkstoffes, z. B. in Ethanol, Propandiol oder Glycerin oder in Gemischen der vorgenannten Lösungsmittel in Frage.

Im folgenden wird die Wirkung der Substanz in Standardtestmethoden beipielhaft erläutert.

Beispiel 1

Stimulierung koloniebildender Zellen des Knochenmarks durch Deoxyspergualin.

Weibliche B6D2FI-Mäuse wurden mit den in Tabelle 1 angegebenen Konzentrationen an 15-Deoxyspergualin behandelt. 15-Deoxyspergualin wurde an 5 aufeinanderfolgenden Tagen intraperitoneal appliziert. 7 Tage nach der ersten Behandlung mit 15-Deoxyspergualin wurde das Knochenmark aus den oberschenkeln der getöteten Mäuse entnommen und die Auswahlzellen mit der Fähigkeit Kolonien zu bilden bestimmt. Dazu wurde die von Stanley et al. (J. Exp. Med. 143, 631(1976)) beschriebene Methode der Softagar-Technik benutzt. Die Tabelle 1 zeigt eine dosisabhängige Stimulation der koloniebildenden Zellen im Knochenmark von 15-Deoxypergualin behandelten Tieren.

TABELLE  1

Stimulation  der  Kolonie-bildenden  Zellen  des  Knochenmarks
durch  15-Deoxyspergualin  in  der  Maus

| Behandlung | Kolonie-bildende Zellen in % der Kontrolle |
|---|---|
| 0  mg/kg  (Kontrolle) | 100 |
| 1  mg/kg | 249 |
| 3  mg/kg | 252 |
| 5  mg/kg | 277 |
| 7  mg/kg | 288 |
| 10  mg/kg | 395 |

Beispiel 2

Therapeutische Behandlung der Experimentellen Allergischen Enzephalomyelitis (EAE) durch 15-Deoxyspergualin.

Die EAE wurde in weiblichen Lewis Ratten durch die Gabe von Meerschweinchen-Rückenmark, kompletten Freund'schen Adjuvans und abgetöteten Bordetella pertussis Keimen induziert. 15-Deoxyspergualin wurde nach Ausbruch der Erkrankung, nämlich 8 Tage nach der Induktion, in einer Konzentration von 0,16 bis 2,5 mg/kg Körpergewicht an fünf aufeinanderfolgenden Tagen entweder peroral oder intraperitoneal verabreicht.

Die Tabelle 2 zeigt, daß in der unbehandelten Kontrollgruppe die Erkrankung zum Tod aller Tiere führt. Die mittlere Überlebenszeit lag bei 15 bis 16 Tagen.

Dosis-abhängig führt die Gabe von 15-Deoxyspergualin zu einer Reduktion der Mortalität und zu einer Heilung der Tiere. 15-Deoxyspergualin ist also in der Lage, die bereits ausgebrochene Erkrankung therapeutisch zu beeinflussen und zwar nicht nur im Sinne einer Verlängerung der Überlebenszeit, sondern auch im Sinne einer Heilung. Geheilte Tiere zeigten keinen Rückfall in ihrer Erkrankung.

TABELLE 2

Therapie der EAE in Lewis Ratten durch 15-Deoxyspergualin

|  | peroral |  | intraperitoneal |  |
|---|---|---|---|---|
| mg/kg | Mortalität |  | mg/kg | Mortalität |
| 0 | 5/5 |  | 0 | 5/5 |
| 0,16 | 3/5 |  | 0,65 | 2/5 |
| 0,32 | 2/5 |  | 1,25 | 0/5 |
| 0,65 | 2/5 |  | 2,50 | 0/5 |
| 1,25 | 1/5 |  |  |  |
| 2,50 | 1/5 |  |  |  |

Im folgenden wird gezeigt, daß die Konzentrationen von 15-Deoxyspergualin, die bei der EAE therapeutisch gewirkt haben, nicht immunsuppressiv wirkten und zu keiner Erhöhung der Infektanfälligkeit führten. Dazu wurden Ratten mit 2,5 mg/kg 15-Deoxyspergualin (peroral oder intraperitoneal) vorbehandelt und anschließend mit Listeria monocytogenes oder Klebsiella pneumoniae infiziert. Tabelle 3 zeigt, daß keine der mit 2,5 mg 15-Deoxyspergualin behandelten Tiere gestorben waren.

Eine Erhöhung der Dosis von 15-Deoxyspergualin in einen Bereich der bekanntermaßen immunsuppressiv ist (5 mg/kg), führt gleichermaßen zu einer Erhöhung der Infektanfälligkeit.

TABELLE 3

Wirkung von 15-Deoxyspergualin auf die Infektionsanfälligkeit von Ratten

|  |  | Mortalität |
|---|---|---|
| Substanz | Klebsiella pneumoniae | Listeria monocytogenes |
| 15-Deoxyspergualin |  |  |
| 2,5 mg/kg, i.p. | 0/10 | 0/10 |
| 5,0 mg/kg, i.p. | 1/10 | 2/10 |
| 15-Deoxyspergualin |  |  |
| 2,5 mg/kg, i.o. | 0/10 | nicht bestimmt |
| 5,0 mg/kg, i.o. | 0/10 | nicht bestimmt |

**Patentansprüche**

1. Verwendung von 15-Deoxyspergualin zur Herstellung eines Arzneimittels für die therapeutische Behandlung von degenerativen Erkrankungen des Zentralnervensystems.

2. Verwendung nach Anspruch 1 zur Herstellung eines Arzneimittels für die therapeutische Behandlung der multiplen Sklerose.

3. Verwendung von 15-Deoxyspergualin nach Anspruch 1 in der stereoisomeren Minus-Form.

**Claims**

1. The use of 15-deoxyspergualine for the preparation of a pharmaceutical for the therapeutic treatment of degenerative diseases of the central nervous system.

2. The use as claimed in claim 1 for the preparation of a pharmaceutical for the therapeutic treatment of multiple sclerosis.

3. The use of 15-deoxyspergualine as claimed in claim 1 in the form of the minus stereoisomer.

**Revendications**

1. Utilisation de la 15-déoxyspergualine pour la fabrication d'un médicament destiné au traitement thérapeutique de maladies dégénératives du système nerveux central.

2. Utilisation selon la revendication 1, pour la fabrication d'un médicament destiné au traitement thérapeutique de la sclérose en plaques.

3. Utilisation de la 15-déoxyspergualine selon la revendication 1, sous la forme stéréoisomère (-).